# EUROPEAN PATENT SPECIFICATION

(11) **EP 0 692 263 B1**
(45) Date of publication and mention of the grant of the patent: **31.10.2001**
(21) Application number: 95109830.0
(22) Date of filing: 23.06.1995
(51) Int. Cl.: A61L 15/50, A61L 15/26, A61L 15/34

(54) **Method of reducing the coefficient of friction of absorbent products and wax coated products produced thereby**
Verfahren zur Verminderung des Reibungskoeffizienten von absorbierenden Produkten und so erhaltene wachsbeschichtete Produkte
Procédé pour réduire le coefficient de friction de produits absorbants et produits recouverts de cire ainsi obtenus

(30) Priority: 24.06.1994 US 265345
(43) Date of publication of application: 17.01.1996
(73) Proprietor: McNEIL-PPC, INC., Milltown New Jersey 08850 (US)
(72) Inventor: Lichstein, Bernard M., Elizabeth, NJ 070203 (US)
(74) Representative: Groening, Hans Wilhelm, Dipl.-Ing.

(56) References cited:
- EP-A- 0 297 828
- WO-A-90/02542
- GB-A- 1 090 421
- GB-A- 1 431 092

## Description

This invention relates to low friction absorbent products having a high molecular weight waxy substance disposed on a surface thereof. These products are useful in application to materials and products that contact living tissue such as human or animal tissue. The invention also relates to a method of reducing the coefficient of friction of absorbent products comprising fabrics and films by applying a surface coating of a high molecular weight waxy substance. The absorbent products include tampons, sanitary napkins, infant diapers, adult incontinence articles, wound dressings and the like.

### DESCRIPTION OF THE PRIOR ART

Lubricants, including waxes, have been used for many years to coat fibers and fabrics used in absorbent products. Lubricants have also been known for use in coating all or part of the surface of a tampon or tampon applicator. Lubricants have been placed directly on tampons and tampon applicators or their packaging for use immediately before or during the insertion of a tampon.

WO 90/02542 discloses a method of decreasing the fiber release characteristic of a tampon comprising the step of coating the tampon with a layer of beeswax and a tampon having a coating of beeswax applied to the longitudinal circumferential surface thereof.

One example of a lubricated tampon applicator is U.S. Patent No. 3,882,869 (Hanke) which describes a water dispersible tampon applicator composed of water-soluble polymer, water insoluble filler, lubricants, plasticizers, surfactants and antioxidants. Polyethylene oxide polymers and hydroxypropyl cellulose are among the materials listed as useful in these compositions. In addition, POLYOX, a polymer with molecular weights as high as two million is disclosed as a resin useful in manufacturing a thermoplastic tampon applicator by molding and extrusion. However, this reference does not discuss lubricating the tampon itself.

A number of patents have required difficult solutions including action by the user prior to or during the tampon insertion, complex lubrication application patterns or locations, and reliance on the tampon user's physiology to activate the lubricant.

U.S. Patent No. 4,690,671 (Coleman et al.) describes a tampon inserter with a rupturable thermoplastic film containing lubricating fluid, a medicating fluid or a combination lubricant/medicament. Lubricants include polyvinyl alcohol, water soluble sterile jelly and N-vinyl pyrrolidinone. DE 3,148,733 (Vereinigte Papierwerke Schickedanz) describes a protective cover for a tampon that has a container with lubricant at the tampon's front end. The container is punctured to release lubricant as the tampon is ejected.

Patents that suggest the application of a lubricant only to limited portions of tampon surfaces include the following: GB 2,203,949 (Smith & Nephew); DE 3.739,163 (Vereinigte Papierwerke AG); and U.S. Patent Nos. 3,724,465 and 3,821,350 (Duchane). The Duchane patents suggest that a water-insoluble stabilizing adjuvant can be used to prevent the migration of a lubricant from the tip of the tampon. Lubricants that are placed on restricted parts of the tampon, being formulated to prevent their movement onto other parts of the tampon surface as well to the tampon's core do not migrate quickly enough to provide lubrication on the uncoated portions of the tampon's surface. This is especially true during the short time it takes to insert the tampon.

Many of the lubricated tampons described in the prior art actually increase friction due to viscous drag of the liquid and semisolid compositions. In addition, the user may experience discomfort due to the tacky nature of the compositions disclosed in the prior art.

Several patents describe lubricated tampons that depend on the physiology of the user to allow the lubricant to be effective. GB 2,056,283 (Kimberly-Clark) describes a lubricant placed at the base of a tampon. The lubricant is activated by the shearing forces developed upon removal of the tampon. U.S. Patent No. 3,805,785 (Marginet) describes a lubricant composition which requires body temperature to melt it, and GB 1,167,034 (Kimberly-Clark) discloses a composition containing solid particles which dissolve in body fluids to provide lubrication during a user's insertion of a tampon.

These approaches will often fail and will usually perform poorly, because the time available for the user's physiology to increase the lubricity of the tampon is quite short. This is especially true if the available time is only during insertion or removal of the tampon. In addition, menstrual flow is quite variable and unpredictable, and the vaginal tract has rather widely distributed temperature and frictional characteristics. Indeed, clinical and diagnostic literature on normal menstruation describes a wide range of variability in the flow of blood and endometrial tissue in terms of: regularity and duration; amount of flow at any particular time, hour or day; and composition, viscosity and shear characteristics. Therefore, the dependence upon any one or combination of these physiological properties to provide or to enhance lubricity of a tampon is quite risky.

Thus, existing lubricated absorbent products often have one or more of the following faults: (1) requiring action by the user to distribute a lubricant on an absorbent product or its applicator prior to application, (2) ineffective lubrication of uncoated portions of the absorbent product when complex and incomplete coating patterns are used, (3) viscous drag of the lubricant itself which may actually cause reduced lubricity and which may provide a tacky feel to the user, and (4) reliance upon a user's physiological attributes which vary widely among users, e.g., adequate menstrual fluid flow or body heat, to improve lubricity. Therefore, a new lubricated absorbent product is needed which exhibits significant reduction in the coefficient of friction between the absorbent article and tissue which it contacts at normal relative shear speeds experienced during application, wearing and removal, while essentially maintaining normal rate of absorption of fluids and normal absorption capacities.

### SUMMARY OF THE INVENTION

I have found that significant reduction in frictional resistance of fabric or films used in absorbent articles may be obtained by applying a small but effective amount of a high molecular weight solid waxy substance to the surface of such fabrics or films, or to the fibers that compose such fabrics. Without the coating, these surfaces can cause irritation when they contact and move along the skin. Products such as tampons, sanitary napkins, infant diapers, and incontinence briefs can be improved by adding a small but effective amount of a waxy coating to their body-contacting surfaces.

The waxy substances which are effective in the products of this invention are preferably high molecular weight, solid waxy substances which have softening points above body temperature. More preferably, the waxy substances are waxy polymeric substances including polyethylene glycols, methoxy polyethylene glycols and polyethylene oxides which remain solid at body temperature. Surprisingly, these solid waxy substances exhibit decreasing frictional resistance with decreasing application levels. The waxy substances may also contain other fatty or waxy materials. The solid waxy substances of this invention may be applied to the surfaces of the fabrics or fibers by procedures known to those of skill in the art.

Preferably, waxy substances should be applied to tampon plugs so as to achieve as low a coefficient of friction as possible with respect to the vaginal wall. This will substantially prevent the abrasion of soft tissue within the vagina during insertion of the tampon and will require only a small ejection force from the user to insert the tampon in the body. Additionally, the vaginal tissue will be protected during use from any abrasive movement against the vaginal wall or sticking to the vaginal tissue. This reduction of sticking and abrasive movement will also make removal of the tampon easy and atraumatic.

### DETAILED DESCRIPTION OF THE PREFERRED EMBODIMENTS

Absorbent articles of the present invention are useful to reduce irritation of human tissue caused by movement of the surface of an absorbent body along the surface of the human tissue. One example of movement that causes irritation is the chafing that is experienced when wearing external sanitary protection products such as sanitary napkins, panty liners, adult incontinence pads and guards and diapers. Another source of irritation is from insertion, wearing and removal of absorbent articles, such as tampons, within body cavities. In order to reduce this irritation and tissue damage, it is helpful to reduce the coefficient of friction on the surface of the absorbent article to allow it to more easily slide along the tissue. Thus, products having a dry, yet lubricous body-contacting surface should reduce irritation and tissue damage. This can be obtained by disposing a high molecular weight waxy substance on the body-contacting surface of the absorbent article. The coating substance is preferably inherently lubricous without requiring activation by moisture or body heat to be an effective lubricant.

There are two types of operating conditions in lubrication: hydrodynamic lubrication and boundary lubrication. Hydrodynamic lubrication occurs with a plentiful supply of lubricant and where the frictional resistance between the bodies depends largely upon the viscosity of the lubricating medium. Hydrodynamic lubrication operates primarily at high speeds, e.g., in high speed machinery, filament winding and textile manufacturing. In this lubrication, the coefficient of friction increases with viscosity and sliding speed and decreases with load. Only the viscosity of the lubricant can be modified as the load and sliding speed are dictated by the process. Since frictional drag is dictated only by the viscosity of the lubricant, thin films of low viscosity are most useful. However, such low viscosity lubricants will migrate over and into absorbent products and, due to viscous drag, will retard the sliding ability of absorbent products, causing discomfort to the user.

Boundary lubrication occurs between surfaces that are slightly lubricated, but where the lubrication supply is insufficient to maintain a hydrodynamic film. This suggests that the coefficient of friction will decrease with high viscosity lubricants and hard waxes. It is also known that the coefficient of friction under boundary lubrication conditions decreases with increasing sliding speed.

I theorize that the operating condition encountered when absorbent products are used on or against human or animal skin and tissue is of the boundary lubrication type. When absorbent products are used on or against human or animal skin and tissue, the magnitude and range of sliding speeds are dictated primarily by the conditions experienced in application, use and removal of the product. Therefore, only the viscosity or hardness of the lubricant can be modified to give maximum lubrication. Indeed, I have found that the higher molecular weight, more viscous lubricants and hard waxes are best at separating these slow speed sliding surfaces, thereby reducing friction.

As noted above, the high molecular weight waxy substances used in the present invention are better able to isolate the sliding surfaces with a minimum of retarding contribution from the viscous drag within the waxy substance itself. This often occurs with lower molecular weight lubricating materials. Thus, it is believed that, in a series of chemically homologous substances, the more viscous materials are better in reducing the coefficient of friction, and therefore, higher molecular weight waxes are more effective than lower molecular weight waxes. For a series of chemically similar waxy solids, the decrease in coefficient of friction will generally continue until a minimum coefficient of friction is obtained at some maximum molecular weight. Thereafter at higher molecular weights, the coefficient of friction will drop only slightly as the materials become more resin-like.

As the coating weight of the waxy substances is increased, the coefficient of friction begins to increase. This increase in the coefficient of friction is most likely due to the frictional drag of the bulk waxy substance at the higher coating weight. The coating weight should not be so great as to substantially reduce the absorbent properties of the absorbent article. At the other extreme, there is a limit to the effectiveness of any of the polymers as the surface coating weight continues to be lowered and the surface of the substrate becomes less efficiently covered with the waxy substance. Therefore, application loads which are too low can result in an insignificant decrease in the coefficient of friction of the body-contacting surface.

### Absorbent Article

Absorbent articles which come into intimate contact with tissue include bandages and wound dressings, sanitary napkins and tampons, and diapers and adult incontinence devices. During application of the absorbent articles or during use, the body-contacting surface can move along the surface of the tissue. In particular, externally worn products, such as sanitary napkins, diapers, and incontinence devices, can promote chafing as they are constantly being moved by the body parts against which they are worn. Positioning features are being introduced to these devices to restrict their movement with and within a user's clothing. Some of these features increase the stiffness of the absorbent product and can interfere with the comfortable movement of the parts of the body which it touches. Without adequate lubrication at the interface between the absorbent article and the wearer's skin, uncomfortable chafing can result. Even when the body contacting surface does not move with respect to the body tissue, the body contacting surface can stick to the tissue which may lead to discomfort or irritation when removed. This may be especially true in the use of tampons. In addition, tampons are inserted into a body cavity and can damage delicate vaginal tissue during this insertion and subsequent removal.

The absorbent articles of the present invention generally comprise an absorbent core and a body-contacting surface. The body-contacting surface may be the outer surface of the absorbent core, or it may be a covering layer comprising a separate fabric or film which is laminated to the absorbent core by heat, adhesive, and the like. Covering layers are generally designed to provide a soft body-contacting surface for the absorbent article and to contain absorbent material within the absorbent core. The body-contacting surface, therefore, may be a covering fabric or film, or it may simply be the surface of a batt of absorbent fibers. Covering fabrics may be woven textiles such as gauze and the like, nonwoven textiles such as adhesively bonded carded webs, thermobonded fabrics, card-and-bind fabrics, spunbonded fabrics, meltblown fabrics, hydroentangled fabrics and needlepunched fabrics, or knitted textiles. Preferred covering fabrics include nonwoven textiles such as those which are traditionally used as covers for diapers, sanitary napkins, incontinence devices, and the like. The covering fabrics may also include laminates or combinations of several of the above-listed fabrics. Covering films also may be apertured polymeric films which are now being used more widely in the sanitary protection field. Representative, non-limiting apertured polymeric films include those manufactured from polyolefins such as polyethylene and polypropylene, polyesters, nylons, or laminates of such films. The apertures can be two- or three-dimensional. The end use of the absorbent article generally will dictate whether the body-contacting surface is a part of the absorbent batt or whether it is a woven, nonwoven or knitted fabric, or a polymeric film.

The body-contacting surface of the absorbent core may be prepared from hydrophilic or hydrophobic materials. Useful hydrophilic materials include cellulosic materials such as rayon fibers and fabrics, cotton fibers and fabrics, and hydrophilic nylon fibers and fabrics. Useful hydrophobic materials include polyolefins such as polyethylene and polypropylene, polyesters and hydrophobic nylons, and polyurethanes. The hydrophobic materials can be in the form of fibers, fabrics, and films. Of course, it is well recognized in the art that hydrophobic materials can be made hydrophilic by incorporating surfactants into the formulation or by treatment with surfactants or high energy discharges such as corona discharge. Again, the end use of the absorbent article generally will dictate the choice of material for the body-contacting surface.

### Waxy Coating

In the practice of the present invention, at least the body-contacting surface of the absorbent article has a minimal waxy coating disposed thereon. The waxy substance is applied in an amount sufficient to reduce the coefficient of friction of the body-contacting surface by at least about 20%, preferably at least about 40%.

The waxy substance is a solid material, preferably having a softening point above body temperature. The waxy substance may be a high molecular weight material. Useful waxy substances include animal wax, such as spermaceti wax; vegetable waxes, such as bayberry, ouricury, palm, rice bran, candelilla and carnauba wax; mineral waxes, such as paraffin, Fischer-Tropsch, ozokerite, ceresine, montan and microcrystalline wax; and synthetic waxes or other waxy synthetic resins, such as high molecular weight polyethylene glycols, chlorinated paraffins, and the like.

By the use of the term wax and waxy, I mean substances which are solid at the body temperature of the tissue to which the absorbent article will be applied. However, it is preferred that the waxy substance is not tacky or greasy. It is also preferred that the waxy substance be inert or nontoxic to the user. Due to their commercial availability and low toxicity, preferred waxy substances include synthetic waxes or waxy resins such as high molecular weight waxy polyolefin oxides. Most preferred waxy substances include high molecular weight polyethylene glycols and methoxy polyethylene glycols which are commercially available in the CARBOWAX series of products from Union Carbide Corp. and high molecular weight polyethylene oxides having terminal hydroxyl groups which are commercially available in the POLYOX series from Union Carbide Corp. It is preferred that the waxy substances used in the practice of the present invention have properties that are conducive to operating under boundary lubrication conditions.

The molecular weight of the waxy substance may vary depending upon the particular waxy substance selected. The minimum molecular weight at which a given type of material is solid at human body temperatures will depend, in addition to its molecular weight, on such factors such as chemical structure, the presence and degree of crosslinking, the presence and degree of branching, and the type and amount of other substances added in the lubricant formulation. The polyethylene glycols available under the CARBOWAX designation preferably have a molecular weight of at least about 1,500, and more preferably, at least about 3,000. The waxy materials generally have a molecular weight of less than about 1,000,000, and a preferred operating range for molecular weight is between about 3,000 to 600,000, and more preferably about 5,000 to 400,000. When selecting between two waxy substances in a chemically homologous series, it is usually preferred that the coating used have the higher molecular weight. While a waxy substance having a molecular weight of the greater than 600,000 may be used, generally substances having higher molecular weights exhibit properties more like resins than waxes. At the other extreme, a waxy substance having a molecular weight less than about 1,500 may be used, but such substances generally are too soft for use in reducing the coefficient of friction of the body-contacting surface to the desired degree. In fact, low molecular weight substances can increase the coefficient of friction by imparting frictional drag.

The waxy substance may be applied to the absorbent article at relatively low coating weights, that is, less than about 5 wt-% (unless otherwise specified, the use of "wt-%" relates the weight of the waxy substance to the total weight of the cover or covering surface of the absorbent article), and more preferably at less than about 3 wt-%. This coating should not substantially retard the permeability of the substrate to liquids that the absorbent product commonly encounters in use and, therefore, should not substantially limit the breathability and absorbency characteristics of the absorbent product. The coating can cover the entire surface, such as the entire fabric, film or fibers which make up the surface. The coating need not be restricted to a particular region of the product surface such as the tip, bottom or segments of the surface, such as spots or strips. Preferably, the waxy substance is present at about 0.05 to 2 wt-%, and more preferably at about 1 to 1.5 wt-%. Greater application loads can increase the cost of the absorbent article can reduce the absorptive capacity and rate of liquid uptake of the absorbent coating.

The coating formulation can also contain additional components such as combinations of the waxy substances described above. The formulations can also contain fatty acids, such as stearic acid and lauric acid; fatty acid esters, such as glycerol stearates, glycerol laurates, methyl myristate and isopropyl palmitate; fatty acid salts, such as magnesium or calcium stearate; silicones, such as dimethylpolysiloxanes; and alcohol alkyl ethers of cellulose, such as hydroxypropyl cellulose. The additional components are employed from 10 to 90 wt-% of the coating formulation. These additional components can be added to modify the function and esthetics of the formulation to make it most suitable for each product and the physiological environment in which it is used. Fillers such as titanium dioxide, talc, silica kaolin, starch and calcium carbonate can also be used to reduce costs and to decrease the greasiness and tackiness of the formulation.

### Method of Making

The absorbent articles of the present invention can be prepared by forming an absorbent article having a body-contacting surface, selecting a waxy substance having a molecular weight of at least about 1,500 and a softening point above human body temperature, and applying an effective amount of the wax to the body-contacting surface to reduce the coefficient of friction of the body-contacting surface by at least about 20%. The waxy substance may be applied to the body-contacting surface before, during, or after the fabrication of the absorbent article. If it is desired to apply the waxy substance before or during the manufacture of the article, it can be applied to fibers which will ultimately make up the entire absorbent core of the absorbent article, only to fibers which will make up the body-contacting surface thereof, or applied to a body-contacting covering material before it is attached to an absorbent core to make the absorbent article. When the waxy substance is applied to the completed absorbent article, it can be controlled to only cover the body-contacting surfaces of the product.

Application of the waxy substance before or during the manufacture of the absorbent article can be done in any of a number of ways known to those of ordinary skill in the art including spraying unassembled fibers; or treating a batt of fibers or a fabric or apertured film by means such as spraying, foaming, dipping, roll coating (including without limitation reverse roll, gravure, transfer roll, roll-over-roll, hot melt roll coating, and the like), vacuum coating, blade coating, hot melt slot coating, etc. The waxy substance will generally by applied in liquid form, either coated or sprayed from the melt or dispersed (including, without limitation, solutions, dispersions, and emulsions) in a liquid carrier such as a suitable organic or aqueous solvent. However, application of the waxy substance in powder form can also be used, especially if the powder is applied at temperatures sufficiently elevated so the powder surface is tacky or soft. Application of the waxy substance to a completed absorbent article can also be accomplished by spraying, dipping, foaming, roll coating, blade application, and the like.

### Method of Use

The waxy substances can be applied to any absorbent article which is intended to be used in contact with human or animal tissue. Non-limiting, representative examples of such uses include tampons, sanitary napkins and panty liners, diapers and incontinence devices, bandages, and the like. The friction reducing effects can be helpful to provide a lubricous, non-chafing and non-sticking surface to the body-contacting cover of a sanitary napkin and/or to the flaps or tabs that are used to position and attach the napkin to a user's undergarment. The friction reducing effects may also be helpful in reducing chafing, irritation and adherence to tissue occurring during the use of diapers, incontinence devices, etc. An important use of the present invention is to provide a catamenial tampon that is easily inserted and removed by virtue of the lubricous coating on the outer surfaces of a tampon. The waxy substances can be applied predominantly to the surface of the tampon, for example, the cover if present or directly to the fibers on the outer surface of the tampon. The waxy substances are not dependent on the user's physiology for their effectiveness. They do not require body heat nor the application of body fluid or other external fluids to cause the wax to be lubricous.

The following example is intended to illustrate the invention but is not intended to limit the invention in any way. The invention is not restricted to either water soluble or water insoluble lubricants. Both types will work in this invention.

### EXAMPLE

The substrate on which coefficient of friction measurements were made was a non-woven fabric, made of a fusible bicomponent fiber comprised of a polyethylene sheath and a polyester core. The fabric was coated with the various formulations from aqueous solutions and/or emulsions using a laboratory padder. Each side of the coated fabrics was cured for 15 seconds at 225° F.

Coefficient of friction measurements were made using a Friction Evaluation Device which moves a cylindrical probe, with a preset load value and speed, over the surface to be measured. The frictional drag of the surface imparts a torque to the cylindrical probe. The probe contains a force gauge transducer that translates torque values into force measurements. The probe can be covered with any material that is appropriate to simulate the use conditions of the substrate being tested. We chose to use a lightly moistened (by touching it with wet tissue) cross-linked collagen film that was free of processing lubricant such as mineral oil.

The coating materials tested were glyceryl monolaurate and an increasing molecular weight series of solid (at 37° C) Carbowax and Polyox polymers. The results can be seen in Tables 1 to 3.

**TABLE 1**

| EFFECT OF LUBRICANTS AT 1.3% ADD-ON IN REDUCING THE COEFFICIENT OF FRICTION | | | |
|---|---|---|---|
| Coating at 1.3% Add-on Material | | Coefficient of Friction | |
| | M.W. | Coef. of Friction | Percent Reduction From Uncoated Fabric |
| Uncoated Fabric | - | 0.437 | - |
| Glyceryl Monolaurate | 274 | 0.354 | 20.0 |
| Polyox N 10 | 100,000 | 0.330 | 24.5 |
| Polyox N 300 | 400,000 | 0.258 | 40.9 |
| Carbowax 3350 | 3000-3700 | 0.240 | 45.1 |
| Carbowax 20M | 5,000-20,000 | 0.137 | 68.6 |

**TABLE 2**

| EFFECT OF VARIOUS LUBRICANTS AT DIFFERENT ADD-ONS IN REDUCING THE COEFFICIENT OF FRICTION | | | |
|---|---|---|---|
| Coating | M.W. | Add-on (%) | COF |
| Untreated Fabric | - | - | 0.437 |
| Glyceryl Monolaurate | 274 | 1.0 | 0.366 |
| Glyceryl Monolaurate | 274 | 2.0 | 0.327 |
| Carbowax 1450 | 1300-1600 | 1.9 | 0.364 |
| Carbowax 3350 | 3000-3700 | 1.2 | 0.223 |
| Carbowax 3350 | 3000-3700 | 1.8 | 0.333 |
| Carbowax 4600 | 4400-4800 | 1.8 | 0.340 |
| Carbowax 8000 | 7000-9000 | 1.7 | 0.330 |
| Carbowax 20M | 15,000-20,000 | 1.3 | 0.137 |
| Carbowax 20M | 15,000-20,000 | 2.0 | 0.191 |
| Polyox N 10 | 100,000 | 1.38 | 0.324 |
| Polyox N 10 | 100,000 | 1.5 | 0.298 |
| Polyox N 10 | 100,000 | 3.26 | 0.254 |
| Polyox N 3000 | 400,000 | 1.5 | 0.242 |
| Polyox N 3000 | 400,000 | 1.85 | 0.221 |

**TABLE 3**

| COEFFICIENT OF FRICTION OF FORMULATIONS CONTAINING CARBOWAX 20M, GLYCERYL MONOLAURATE AND SILICONE | | | |
|---|---|---|---|
| Carbowax 20M | Glyceryl Monolaurate | Silicone | COF |
| - | - | - | 0.437 |
| 0.25 | - | - | 0.255 |
| 0.37 | - | - | 0.161 |
| 0.5 | - | - | 0.188 |
| 1.0 | - | - | 0.164 |
| 1.3 | - | - | 0.137 |
| 2.0 | - | - | 0.191 |
| 1.0 | 1.0 | - | 0.176 |
| 0.66 | 1.0 | - | 0.185 |
| 0.33 | 1.0 | - | 0.251 |
| 0.25 | 1.0 | - | 0.256 |
| 0.5 | - | 0.1 | 0.193 |
| 0.5 | 1.0 | 0.1 | 0.191 |
| - | - | 0.1 | 0.335 |

Significant reductions of coefficient of friction were seen for all coating materials when compared with uncoated fabric. The reduction of coefficient of friction was generally best for Carbowax polymers and Polyox polymers, followed by glyceryl monolaurate. For both the Polyox and Carbowax polymers the reduction of coefficient of friction generally increased with increasing molecular weight within each polymer series, as shown in Table 2. Comparing, where possible at the same add-on of about 1.3%, the reduction of coefficient of friction, 69%, was best for the highest molecular weight Carbowax 20M (M.W. 15,000 to 20,000) when compared to the untreated fabric. The reduction of coefficient of friction was 41% for the highest molecular weight Polyox (M.W. 400,000) and 20% for glyceryl monolaurate (M.W. 274). All these materials melt at about 60 to 65° C.

An additional observation is that the coefficient of friction decreased with decreasing add-on for the Carbowax polymers indicating a minimum addition of about 1.3% by weight for optimum reduction in the coefficient of friction. A plot of the Carbowax 20M data, Table 3, would indicate a minimum coefficient of friction at about 1.3% by weight of polymer.

Significant reductions in coefficient of friction were obtained even at 0.25%, the lowest add-on tested. It is anticipated that significant reductions in coefficient of friction will be obtained at add-ons as low as 0.05%.

Excellent reduction in the coefficient of friction is obtained at added polymer amounts up to 5% of the substrate, preferably from 0.05% to 2%, and most preferably from 1% to 2% added waxy polymer.

The specification and examples above are presented to aid in the complete and non-limiting understanding of the invention disclosed herein. Since many variations and embodiments of the invention can be made without departing from its spirit and scope, the invention resides in the claims hereinafter appended.

## Claims

1. An absorbent article, comprising:
(a) an absorbent core comprising a fibrous, fluid-absorbing material and having a body-contacting surface;
(b) an effective, friction-reducing amount of a waxy substance other than beeswax and having a molecular weight of at least about 1,500 and a softening point above human body temperature disposed on the body-contacting surface.

2. The absorbent article of claim 1 wherein the body-contacting surface comprises a plurality of fibers.

3. The absorbent article of claim 2 wherein the body-contacting surface comprises a fabric cover.

4. The absorbent article of claim 3 wherein the fabric cover is a nonwoven fabric.

5. The absorbent article of claim 1 wherein the body-contacting surface comprises a polymeric film cover

6. The absorbent article of claim 1 wherein the wax has a molecular weight of about 1,500 to 1,000,000.

7. The absorbent article of claim 1 wherein the wax is a polymeric wax.

8. The absorbent article of claim 1 wherein the wax is present at about 0.05 to 5 wt-%.

9. The absorbent article of claim 1 which is a tampon.

10. A method for reducing chafing or irritation of human tissue which is caused by contact with an absorbent article having a body-contacting surface having a coefficient of friction, the method comprising the steps of: selecting a waxy substance having a molecular weight of at least about 1,500 and a softening point above human body temperature and applying an effective amount of the wax to the body-contacting surface to reduce the coefficient of friction of the body-contacting surface by at least about 20%.

11. The method of claim 10 wherein the wax has a molecular weight of about 1,500 to 1,000,000.

12. The method of claim 10 wherein the wax is a polymeric wax.

## Patentansprüche

1. Absorbierender Gegenstand, umfassend:
(a) einen absorbierenden Kern, welcher ein faserartiges, fluidabsorbierendes Material umfaßt und eine körperberührende Oberfläche aufweist;
(b) eine wirksame, reibungvermindernde Menge einer wachsartigen Substanz, außer Bienenwachs, mit einem Molekulargewicht von wenigstens etwa 1500 und einem Erweichungspunkt oberhalb von menschlicher Körpertemperatur, welche auf der körperberührenden Oberfläche abgeschieden ist.

2. Absorbierender Gegenstand nach Anspruch 1, **dadurch gekennzeichnet, daß** die körperberührende Oberfläche eine Vielzahl an Fasern umfaßt.

3. Absorbierender Gegenstand nach Anspruch 2, **dadurch gekennzeichnet, daß** die körperberührende Oberfläche eine Gewebebedeckung umfaßt.

4. Absorbierender Gegenstand nach Anspruch 3, **dadurch gekennzeichnet, daß** die Gewebebedeckung ein Vliesstoff ist.

5. Absorbierender Gegenstand nach Anspruch 1, **dadurch gekennzeichnet, daß** die körperberührende Oberfläche eine Polymerfilmbedeckung umfaßt.

6. Absorbierender Gegenstand nach Anspruch 1, **dadurch gekennzeichnet, daß** das Wachs ein Molekulargewicht von etwa 1.500 bis 1.000.000 aufweist.

7. Absorbierender Gegenstand nach Anspruch 1, **dadurch gekennzeichnet, daß** das Wachs ein polymeres Wachs ist.

8. Absorbierender Gegenstand nach Anspruch 1, **dadurch gekennzeichnet, daß** das Wachs mit etwa 0,05 bis 5 Gewichtsprozent vorhanden ist.

9. Absorbierender Gegenstand nach Anspruch 1, welcher ein Tampon ist.

10. Verfahren zum Reduzieren eines Scheuerns oder einer Irritation von menschlichem Gewebe, welches bzw. welche bewirkt wird durch Berührung mit einem absorbierenden Gegenstand, der eine körperberührende Oberfläche mit einem Reibungskoeffizienten aufweist, wobei das Verfahren die Schritte umfaßt: Auswählen einer wachsartigen Substanz mit einem Molekulargewicht von wenigstens etwa 1500 und einem Erweichungspunkt oberhalb von menschlicher Körpertemperatur und Auftragen einer wirksamen Menge des Wachses auf die körperberührende Oberfläche, um den Reibungskoeffizienten der körperberührenden Oberfläche um wenigstens etwa 20 % zu reduzieren.

11. Verfahren nach Anspruch 10, **dadurch gekennzeichnet, daß** das Wachs ein Molekulargewicht von etwa 1500 bis 1000000 aufweist.

12. Verfahren nach Anspruch 10, **dadurch gekennzeichnet, daß** das Wachs ein polymeres Wachs ist.

## Revendications

1. Article absorbant, comprenant :
(a) une partie centrale comprenant un matériau fibreux absorbant les fluides et ayant une surface en contact avec le corps ;
(b) une quantité efficace réduisant le frottement d'une substance cireuse autre que la cire d'abeilles et ayant une masse moléculaire d'au moins environ 1 500 et un point de ramollissement supérieur à la température corporelle humaine disposée sur la surface en contact avec le corps.

2. Article absorbant selon la revendication 1, dans lequel la surface en contact avec le corps comprend une pluralité de fibres.

3. Article absorbant selon la revendication 2, dans lequel la surface en contact avec le corps comprend un revêtement en tissu.

4. Article absorbant selon la revendication 3, dans lequel le revêtement en tissu est un tissu non tissé.

5. Article absorbant selon la revendication 1, dans lequel la surface en contact avec le corps comprend un revêtement en film polymère.

6. Article absorbant selon la revendication 1, dans lequel la cire a une masse moléculaire d'environ 1 500 à 1 000 000.

7. Article absorbant selon la revendication 1, dans lequel la cire est une cire polymère.

8. Article absorbant selon la revendication 1, dans lequel la cire est présente à raison d'environ 0,05 à 5% en poids.

9. Article absorbant selon la revendication 1, lequel est un tampon.

10. Procédé pour réduire le frottement ou l'irritation d'un tissu humain résultant d'un contact entre un article absorbant ayant une surface en contact avec le corps présentant un coefficient de friction, le procédé comprenant les étapes consistant à : sélectionner une substance cireuse ayant une masse moléculaire d'au moins environ 1500 et un point de ramollissement supérieur à la température corporelle humaine et à appliquer une quantité efficace de la cire sur la surface en contact avec le corps afin de réduire le coefficient de friction de la surface en contact avec le corps d'au moins environ 20 %.

11. Procédé selon la revendication 10, dans lequel la cire a une masse moléculaire comprise entre environ 1500 et 1 000 000.

12. Procédé selon la revendication 10, dans lequel la cire est une cire polymère.
